(19) Europäisches Patentamt
European Patent Office
Office européen des brevets

(11) Veröffentlichungsnummer: **0 011 121**
**B1**

(12) # EUROPÄISCHE PATENTSCHRIFT

(45) Veröffentlichungstag der Patentschrift: **05.05.82**

(51) Int. Cl.³: **C 07 J 5/00,** A 61 K 31/57

(21) Anmeldenummer: **79103778.1**

(22) Anmeldetag: **03.10.79**

(54) **Hydrocortisonester, diese enthaltende pharmazeutische Zubereitungen und Verfahren zu ihrer Herstellung.**

(30) Priorität: **08.11.78 DE 2848423**

(43) Veröffentlichungstag der Anmeldung: **28.05.80 Patentblatt 80/11**

(45) Bekanntmachung des Hinweises auf die Patenterteilung: **05.05.82 Patentblatt 82/18**

(84) Benannte Vertragsstaaten: **AT BE CH DE FR GB IT NL SE**

(56) Entgegenhaltungen:
**GB-A-1 007 925**

(73) Patentinhaber: **Merck Patent Gesellschaft mit beschränkter Haftung, Frankfurter Strasse 250, D-6100 Darmstadt (DE)**

(72) Erfinder: **Baumgarth, Manfred, Dr., Sachsenstrasse 53, D-6100 Darmstadt (DE)**
Erfinder: **Orth, Dieter, Dr., Jahnstrasse 83, D-6100 Darmstadt (DE)**
Erfinder: **Harting, Jürgen, Dr., Heidelbergerstrasse 123, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Hydrocortisonester, diese enthaltende pharmazeutische Zubereitungen und Verfahren zu ihrer Herstellung

Die Erfindung betrifft neue Hydrocortisonester der allgemeinen Formel I

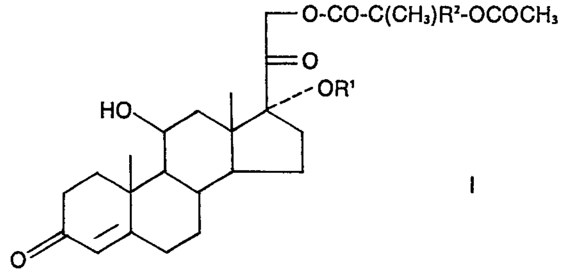

I

worin $R^1$ jeweils H, Formyl oder Acetyl und
$R^2$ jeweils H oder $CH_3$
bedeuten.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, dass die Verbindungen der Formel I bei guter Verträglichkeit wertvolle pharmakologische Eigenschaften besitzen. Insbesondere treten antiphlogistische Wirkungen auf, die sich z.B. auf eine antiproliferative Wirkungskomponente [feststellbar z.B. analog der Methodik von Rudas, Drug Research 10, 226 (1969)], eine antiexsudative Wirkungskomponente [feststellbar z.B. analog der von Hotovy und Kapff, Arch. Int. Pharmacodyn. 111, 420 - 436 (1957) beschriebenen Methode; Granulombeuteltest], eine thymolytische Wirkungskomponente [feststellbar z.B. analog der Methodik von Steelman et al., Steroids 1, 163 (1963)], und eine ACTH-beeinflussende Wirkungskomponente [feststellbar aufgrund der Hemmung einer Nebennierenhypertrophie analog der Methodik von Bohus, B., Acta Physiol. Acad. Sci. Hung. 29, 203 (1966)] zurückführen lassen. Daher eignen sich die Verbindungen der Formel I z.B. zur Bekämpfung hartnäckiger Allergien und anderer entzündlicher Erkrankungen der Haut, ferner zur Behandlung rheumatischer Arthritis. Diese Wirkungen sind nach den dafür üblichen Untersuchungsmethoden erfassbar.

Die Verbindungen der Formel I können daher als Arzneimittelwirkstoffe in der Human- und Veterinärmedizin und auch als Zwischenprodukte zur Herstellung anderer Arzneimittelwirkstoffe verwendet werden.

Ähnliche Steroidester sind aus der GB-A-1 007 925 bekannt. So sind dort in allgemeiner Form Ester der Formel St-OOC-$R_1$-OOC-$R_2$ beschrieben, die von Glucocorticoiden der Formel St-OH (wobei die OH-Gruppe in 21-Stellung steht) abgeleitet sind und worin die Reste $R_1$ bzw. $R_2$ so beschaffen sind, dass die Verbindung HO$R_1$COOH eine aliphatische Hydroxycarbonsäure und die Verbindung $R_2$-COOH eine aliphatische oder aromatische Carbonsäure ist. Von den vielen möglichen Verbindungen, die von dieser allgemeinen Formel umschlossen werden, sind jedoch konkret nur einige Prednisolon- und Cortisonester genannt, die den vorliegenden Hydrocortisonestern strukturell ferner stehen als beispielsweise das bekannte Handelspräparat Hydrocortison-21-acetat.

Gegenstand der Erfindung sind die Verbindungen der Formel I. In der Formel I bedeutet der Rest $R^1$ vorzugsweise H oder Acetyl; der Rest $R^2$ bedeutet bevorzugt H. Dementsprechend sind Gegenstand der Erfindung insbesondere diejenigen Verbindungen der Formel I, in denen mindestens einer der Reste $R^1$ und $R^2$ eine der vorstehend angegebenen bevorzugten Bedeutungen hat.

Gegenstand der Erfindung ist ferner ein Verfahren zur Herstellung der Verbindungen der Formel I, dadurch gekennzeichnet, dass man ein Steroid der allgemeinen Formel II

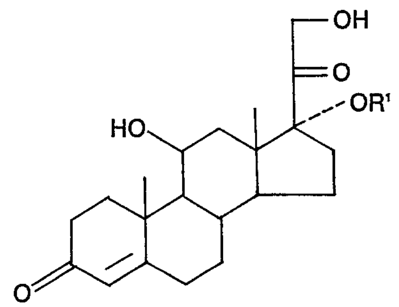

II

worin der Rest $R^1$ die oben angegebene Bedeutung hat, oder ein funktionell abgewandeltes Derivat desselben mit einer 2-Hydroxyfettsäure der allgemeinen Formel III

$$CH_3\text{-}CR^2(OH)\text{-}COOH \qquad III$$

worin der Rest $R^2$ die oben angegebene Bedeutung hat,
oder einem funktionell abgewandelten Derivat derselben umsetzt und dass man, falls im 21-Substituenten eine Hydroxygruppe vorhanden ist, diese acetyliert.

Die Hydrocortison-ester der allgemeinen Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York) beschrieben sind, und zwar unter Reaktionsbedingungen, wie sie für diese Umsetzungen bekannt und geeignet sind. Dabei kann man auch von an sich bekannten, hier nicht näher erwähnten Varianten Gebrauch machen.

Als funktionell abgewandelte Derivate der Steroide der Formel II kommen bevorzugt die entsprechenden 21-Jod-Verbindungen oder die 21-Methansulfonate in Betracht, ferner auch die 21- p-Toluolsulfonate. Funktionell abgewandelte

Derivate der Verbindungen der Formel III sind bevorzugt die entsprechenden Carbonsäurehalogenide, insbesondere die Chloride und Bromide und die entsprechenden Säureanhydride sowie die entsprechenden Derivate mit einer acetylierten Hydroxygruppe an $C_{(2)}$,

Die Verbindungen III können racemisch oder optisch-aktiv sein. Dementsprechend umschliessen die Verbindungen der Formel I bezüglich des asymmetrischen C-Atoms im 21-Substituenten racemische und optisch-aktive Formen.

Die Steroide der Formel II werden vorzugsweise mit Säuren der Formel $CH_3$-$CR^2(OCOCH_3)$-COOH (IIIA) oder deren an der Carboxygruppe funktionell abgewandelten Derivaten, wie z.B. deren Halogeniden (z.B. Chloriden) oder Anhydriden umgesetzt, zweckmässig bei Temperaturen zwischen −100 und +200°, vorzugsweise zwischen etwa −20 und 150°. Die Reaktionszeiten bewegen sich etwa zwischen 1 Minute und 48 Stunden, vorzugsweise zwischen 10 Minuten und 24 Stunden.

Wird als Veresterungsmittel eine der oben genannten Säuren IIIA eingesetzt, so kann man ohne oder unter Zusatz von Katalysatoren, wie Schwefelsäure, Chlorwasserstoff, Phosphorsäure, aromatischen Sulfonsäuren, wie p-Toluolsulfonsäure, oder sauren Ionenaustauschern arbeiten, und zwar vorzugsweise bei Temperaturen zwischen 10 und 150°. Die Säure IIIA wird gewöhnlich im Überschuss eingesetzt.

Man kann auch in Gegenwart von wasserbindenden Agenzien arbeiten, z.B. von Molekularsieben oder von wasserfreien Schwermetallsulfaten, wie Kupfer-, Eisen-, Nickel-, Kobalt- oder Zinksulfat. Man kann auch das bei der Veresterung gebildete Wasser durch azeotrope Destillation entfernen, wobei man vorteilhaft Kohlenwasserstoffe, wie Benzol oder Toluol, oder chlorierte Kohlenwasserstoffe, wie Chloroform oder 1,2-Trichloräthan als inerte organische Lösungsmittel zusetzt.

Unter sehr milden Bedingungen verläuft die Veresterung, wenn man das Reaktionswasser chemisch durch Zusatz von vorzugsweise molekularen Mengen an Carbodiimiden, wie N,N'-Di--cyclohexyl-carbodiimid bindet, wobei man in inerten Lösungsmitteln, wie Äther, Dioxan, Benzol, Äthylenglycoldimethyläther oder bevorzugt in Basen wie Pyridin arbeitet.

Man kann die Steroide der Formel II auch mit funktionell abgewandelten Derivaten der Säuren IIIA, z.B. den Halogeniden oder Anhydriden ohne oder unter Zusatz von säurebindenden Mitteln, z.B. von Natrium- oder Kaliumhydroxid, Natrium- oder Kaliumcarbonat, einer organischen Base wie Pyridin, Chinolin, Collidin oder Triäthylamin umsetzen. Als Lösungsmittel kommen inerte organische wie Äther, Tetrahydrofuran oder Benzol in Frage. Man kann auch die überschüssigen Halogenide oder Anhydride oder einen Überschuss der Base als Lösungsmittel benutzen. Die Veresterung wird zweckmässig bei Temperaturen zwichen −20 und 150, vorzugsweise zwischen 0 und 30° vorgenommen und dauert in der Regel zwischen 10 Minuten und 24 Stunden.

Es ist auch möglich, von funktionell abgewandelten Derivaten der Steroide II auszugehen, insbesondere von entsprechenden 21-Jodsteroiden. Diese sind zugänglich durch Umsetzung der Steroide II mit Methansulfonylchlorid zu den entsprechenden 21-Methansulfonyloxysteroiden und anschliessenden Ersatz der Methansulfonyloxygruppe durch Jod. Die 21-Jodsteroide werden vorzugsweise mit den Carbonsäuren III oder IIIA in Gegenwart von Aminen wie Triäthylamin oder Pyridin umgesetzt, ferner mit den Salzen dieser Carbonsäuren, z.B. den Alkalimetall-, Erdalkalimetall-, Magnesium-, Zink- oder Ammoniumsalzen, bevorzugt den Natrium- oder Kaliumsalzen. Die Umsetzung wird in der Regel in Gegenwart eines inerten Lösungsmittels wie Aceton oder Äther durchgeführt. Die Reaktionstemperaturen liegen zwischen etwa 40 und 120, vorzugsweise 40 und 70°; die Reaktionszeiten liegen zwischen etwa 1 und 15 Stunden, im allgemeinen zwischen 5 und 10 Stunden.

Falls das erhaltene Produkt im 21-Substituenten noch eine freie Hydroxygruppe enthält, wird diese anschliessend nach einer gebräuchlichen schonenden Acetylierungsmethode acetyliert. Das kann z.B. durch Umsetzung mit einem Überschuss Acetanhydrid in Pyridin bei Raumtemperatur geschehen. Bei der Acetylierung wird die — weniger reaktionsfähige — 11-Hydroxygruppe des Hydrocortisons nicht angegriffen.

Gegenstand der Erfindung ist ferner die Verwendung der neuen Verbindungen der Formel I zur Herstellung pharmazeutischer Zubereitungen, insbesondere auf nicht-chemischem Wege. Die Verbindungen I können zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I.

Diese Zubereitungen können als Arzneimittel in der Human- und Veterinärmedizin verwendet werden. Als Trägerstoffe kommen organische oder anorganische Substanzen in Frage, die sich insbesondere für die topische Applikation eignen und mit den neuen Verbindungen nicht reagieren, beispielsweise Wasser, pflanzliche Öle, Kohlenwasserstoffe wie alkylierte Naphthaline, halogenierte Kohlenwasserstoffe wie $CF_2Cl_2$ (z.B. für Aerosole), Benzylalkohole, Polyäthylenglycole, Glycerintriacetat, Gelatine, Kohlehydrate wie Lactose oder Stärke, Magnesiumstearat, Talk, Vaseline®. Zur topischen Anwendung dienen insbesondere Lösungen, Lotionen, Emulsionen, Sprays (Aerosole), Salben, Cremes, Pasten oder Puder. Die neuen Verbindungen können auch lyophilisiert werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Antibiotika wie Gentamycin und/oder Antimykotika und/oder andere topisch wirkende Substanzen.

Die neuen Verbindungen werden in der Regel in Analogie zu bekannten, im Handel befindlichen Entzündungshemmern (z.B. Hydrocortison-17-butyrat) verabreicht. Bei der topischen Applikation in Kombination mit dafür geeigneten Trägerstoffen kann eine gute Aktivität über grössere Verdünnungsbereiche festgestellt werden.

Beispielsweise zeigen sich Konzentrationen des Wirkstoffs zwischen etwa 0,05 und 1 Gewichtsprozent, bezogen auf das Gewicht des verwendeten Präparats, als wirksam zur Heilung von Entzündungen. Bevorzugt sind Konzentrationen von etwa 0,1 bis 0,5 Gewichtsprozent.

Jede der in den folgenden Beispielen genannten Verbindungen der Formel I ist zur Herstellung von pharmazeutischen Zubereitungen besonders geeignet.

Vor- und nachstehend sind die Temperaturen in °C angegeben.

Beispiel 1

Man tropft 11,16 g Acetylmilchsäurechlorid unter Feuchtigkeitsausschluss bei 0 bis 5° zu einer Lösung von 10 g Hydrocortison-17-acetat in 100 ml Pyridin, rührt die Mischung eine Stunde bei 20°, giesst anschliessend in 5%ige eiskalte Salzsäure und extrahiert den Niederschlag mit Dichlormethan. Man wäscht mit Wasser, gesättigter Natriumhydrogencarbonatlösung und Wasser und trocknet über Natriumsulfat. Nach Eindampfen und Chromatographie an Kieselgel (Laufmittel: Dichlormethan/Petroläther/Aceton 10:10:3) erhält man Hydrocortison-17-acetat-21-(2--acetoxypropionat) vom. F. 115 - 117° (aus Äther); $[\alpha]_D^{20} = +64,2°$ ($CHCl_3$).

Beispiele 2 bis 6

Analog Beispiel 1 erhält man aus Hydrocortison, Hydrocortison-17-formiat oder Hydrocortison-17-acetat mit Acetylmilchsäurechlorid oder 2-Acetoxy-isobuttersäurechlorid:

2. Hydrocortison-17-acetat-21-(2-acetoxy-isobutyrat),
   F. 114 - 117° (aus Aceton).
3. Hydrocortison-21-(2-acetoxypropionat),
   F. 182 - 183° (aus Aceton);
   $[\alpha]_D^{20} = +185,1°$ ($CHCl_3$).
4. Hydrocortison-21-(2-acetoxyisobutyrat),
   F. 180 - 181° (aus Aceton/Äther/Petroläther).
5. Hydrocortison-17-formiat-21-(2-acetoxy-propionat).
6. Hydrocortison-17-formiat-21-(2-acetoxy-isobutyrat).

Beispiel 7

188,8 g Hydrocortison-17-acetat in 144 ml Pyridin werden unter Feuchtigkeitsausschluss mit 170 g Acetylmilchsäureanhydrid [Kp. 107° bei 0,01 Torr; herstellbar durch Umsetzung von Michsäure mit Acetylchlorid und anschliessende Behandlung der entstandenen Acetylmilchsäure (Kp. 110° bei 5 Torr) mit Dicyclohexylcarbodiimid in Äther] versetzt und die Mischung 2 Stunden bei 20° stehengelassen. Es wird in Eiswasser gegossen und mit Dichlormethan extrahiert. Man wäscht den Extrakt mit Wasser, 10%iger Salzsäure, gesättigter Natriumhydrogencarbonatlösung und Wasser und trocknet über Natriumsulfat. Nach Eindampfen erhält man Hydrocortison-17-acetat-21-(2-acetoxypropionat), F. 115 bis 117°C (aus Äther); $[\alpha]_D^{20} = +64,2°$ ($CHCl_3$).

Beispiel 8

Man versetzt 4 g Hydrocortison-17-acetat in 80 ml Pyridin mit 8 g Acetylmilchsäure und anschliessend mit 4 g Dicyclohexylcarbodiimid, rührt 20 Stunden unter Feuchtigkeitsausschluss und filtriert das ausgefallene Produkt ab. Das Filtrat wird mit Wasser versetzt, mit verdünnter Salzsäure angesäuert und anschliessend mit Äthylacetat extrahiert. Man wäscht die organische Phase mit Natriumhydrogencarbonatlösung und Wasser, trocknet über Natriumsulfat, dampft ein und erhält Hydrocortison-17-acetat-21-(2--acetoxypropionat); F. 115 - 117° (aus Äther); $[\alpha]_D^{20} = +64,2°$ ($CHCl_3$).

Beispiel 9

Eine Lösung von 23,5 g 21-Deshydroxy-21-jod--hydrocortison (erhalten durch Einwirkung von Methansulfonsäurechlorid auf Hydrocortison und anschliessende Umsetzung des erhaltenen Hydrocortison-21-methansulfonats mit Natriumjodid in Aceton), 6,6 g Acetylmilchsäure und 8,8 ml Triäthylamin in 1000 ml Aceton wird 7 Stunden gekocht und anschliessend auf etwa die Hälfte des Volumens eingedampft. Man giesst in Wasser, neutralisiert und filtriert das erhaltene Hydrocortison-21-(2-acetoxypropionat) ab.

Beispiel 10

Eine Lösung von 23,5 g 21-Deshydroxy-11-jod--hydrocortison, 4,5 g Milchsäure und 8,8 ml Triäthylamin in 1000 ml Aceton wird analog Beispiel 9 umgesetzt und aufgearbeitet. Das rohe Hydrocortison-21-(2-hydroxypropionat) wird anschliessend mit 200 ml trockenem Pyridin und 100 ml Acetanhydrid 3 Stunden bei 25° stehengelassen. Danach rührt man in Eiswasser, isoliert den Niederschlag und erhält Hydrocortison-21-(2-acetoxypropionat).

Die folgenden Beispiele betreffen pharmazeutische Zubereitungen, die Verbindungen der Formel I enthalten (Prozentangaben sind Gewichtsprozente).

Beispiel A: Salbe

| | |
|---|---|
| Hydrocortison-17-acetat-21-(2--acetoxypropionat) | 0,25 % |
| Wollfett wasserfrei | 2,0 % |
| Paraffin dickflüssig | 10,0 % |
| Vaseline® weiss | ad 100,0 % |

Beispiel B: Creme

| | |
|---|---|
| Hydrocortison-17-acetat-21-(2--acetoxypropionat) | 0,5 % |
| Cetylalkohol | 9,0 % |
| Paraffin dickflüssig | 3,0 % |
| Glycerinmonostearat | 2,0 % |
| Propylenglykolmonostearat | 2,0 % |
| Glycerin | 2,0 % |
| feinstteilige Kieselsäure | 0,1 % |
| Vaseline® | 10,0 % |
| Polyoxiäthylensorbitanmonopalmitat | 30,0 % |
| p-Hydroxybenzoesäuremethylester | 0,065% |
| p-Hydroxybenzoesäurepropylester | 0,035% |
| Propylenglykol | 3,0 % |
| Wasser | ad 100,0 % |

Beispiel C: Lotion

| | |
|---|---|
| Hydrocortison-17-acetat-21-(2--acetoxypropionat) | 0,2 % |
| Paraffinöl dickflüssig | 10,0 % |
| Äthanol | 2,0 % |
| Glycerin | 1,0 % |
| Propylenglykol | 2,0 % |
| Sorbinsäure | 0,15 % |
| Fettalkoholpolyglykoläther | 2,0 % |
| Gemisch aus Cetylstearylalkohol und cetylstearylschwefelsaurem Natrium und nichtionogenem Emulgator | 0,5 % |
| Maiglöckchenparfümöl | 0,01 % |
| Wasser | ad 100,0 % |

Beispiel D: Salbe

| | |
|---|---|
| Hydrocortison-17-acetat-21-(2--acetoxypropionat) | 0,1 % |
| Gentamycinsulfat (bezogen auf freie Gentamycinbase) | 0,1 % |
| Cetylalkohol | 2,4 % |
| Wollfett wasserfrei | 1,0 % |
| Paraffin dickflüssig | 15,0 % |
| Vaseline® weiss | ad 100,0 % |

**Patentansprüche für die Vertragsstaaten:**
BE, CH, DE, FR, GB, IT, NL, SE

1. Hydrocortison-ester der allgemeinen Formel I

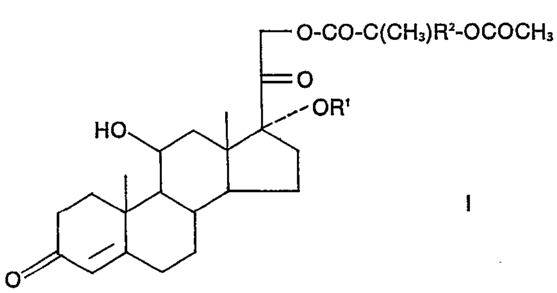

I

worin $R^1$ jeweils H, Formyl oder Acetyl und
$R^2$ jeweils H oder $CH_3$
bedeuten.

2. a) Hydrocortison-21-(2-acetoxypropionat)
b) Hydrocortison-17-acetat-21-(2-acetoxy-propionat)
c) Hydrocortison-21-(2-acetoxyisobutyrat)
d) Hydrocortison-17-acetat-21-(2-acetoxy-isobutyrat).

3. Verfahren zur Herstellung von Hydrocortison-estern der allgemeinen Formel I, dadurch gekennzeichnet, dass man ein Steroid der allgemeinen Formel II

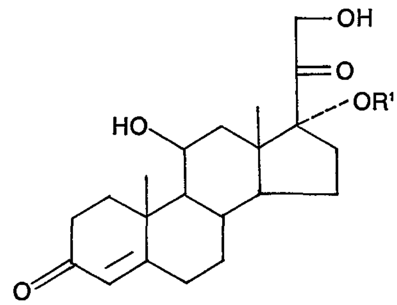

II

worin der Rest $R^1$ die in Anspruch 1 angegebene Bedeutung hat, oder ein funktionell abgewandeltes Derivat desselben mit einer 2-Hydroxyfettsäure der allgemeinen Formel III

$$CH_3\text{-}CR^2(OH)\text{-}COOH \qquad III$$

worin der Rest $R^2$ die in Anspruch 1 angegebene Bedeutung hat, oder einem funktionell abgewandelten Derivat derselben umsetzt und dass man, falls im 21-Substituenten eine Hydroxygruppe vorhanden ist, diese acetyliert.

4. Verfahren zur Herstellung pharmazeutischer Zubereitungen, dadurch gekennzeichnet, dass man eine Verbindung der Formel I zusammen mit mindestens einem festen, flüssigen oder halbflüssigen Träger- oder Hilfsstoff und gegebenenfalls in Kombination mit einem weiteren Wirkstoff in eine geeignete Dosierungsform bringt.

5. Pharmazeutische Zubereitung, gekennzeichnet durch einen Gehalt an mindestens einer Verbindung der allgemeinen Formel I.

**Patentanspruch für den Vertragsstaat:** AT

Verfahren zur Herstellung von Hydrocortisonestern der allgemeinen Formel I

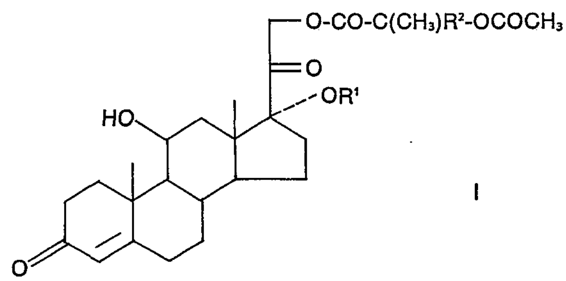

I

worin $R^1$ jeweils H, Formyl oder Acetyl und
$R^2$ jeweils H oder $CH_3$
bedeuten,
dadurch gekennzeichnet, dass man ein Steroid der allgemeinen Formel II

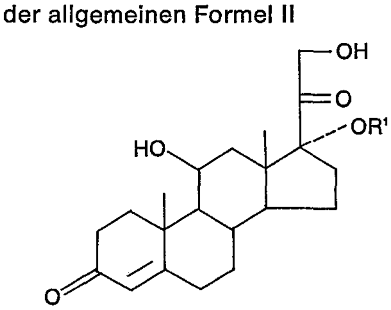

II

worin der Rest $R^1$ die oben angegebene Bedeutung hat, oder ein funktionell abgewandeltes Derivat desselben mit einer 2-Hydroxyfettsäure der allgemeinen Formel III

$CH_3$-$CR^2$(OH)-COOH III

worin der Rest $R^2$ die oben angegebene Bedeutung hat, oder einem funktionell abgewandelten Derivat derselben umsetzt und dass man, falls im 21-Substituenten eine Hydroxygruppe vorhanden ist, diese acetyliert.

**Claims for the Contracting States:**
BE, CH, DE, FR, GB, IT, NL, SE

1. Hydrocortisone esters of the general formula I

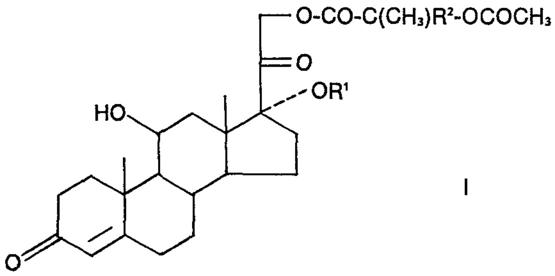

I

in which $R^1$ in each case is H, formyl or acetyl and $R^2$ in each case is H or $CH_3$.

2. a) Hydrocortisone 21-(2-acetoxypropionate)
   b) Hydrocortisone 17-acetate 21-(2-acetoxypropionate)
   c) Hydrocortisone 21-(2-acetoxyisobutyrate)
   d) Hydrocortisone 17-acetate 21-(2-acetoxyisobutyrate).

3. Process for the preparation of hydrocortisone esters of the general formula I, characterised in that a steroid of the general formula II

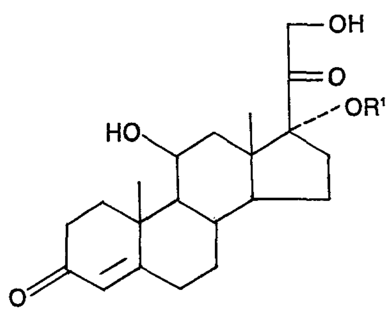

II

in which the radical $R^1$ is as defined in claim 1, or a functionally modified derivative thereof, is reacted with a 2-hydroxy-fatty acid of the general formula III

$CH_3$-$CR^2$(OH)-COOH III

in which the radical $R^2$ is as defined in claim 1, or with a functionally modified derivative thereof, and in that, if a hydroxyl group is present in the substituent in the 21-position, this group is acetylated.

4. Process for the preparation of pharmaceutical formulations, characterised in that a compound of the formula I is brought into a suitable dosage form together with at least one solid, liquid or semi-liquid excipient or auxiliary and optionally in combination with a further active compound.

5. Pharmaceutical formulation, characterised in that it contains at least one compound of the general formula I.

**Claim for the Contracting State:** AT

Process for the preparation of hydrocortisone esters of the general formula I

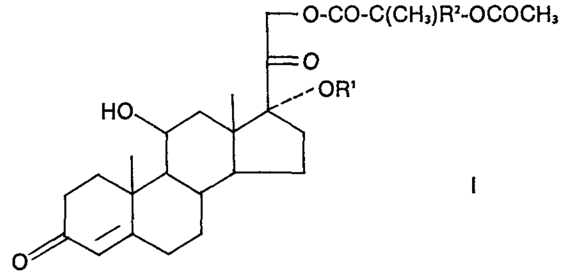

I

in which $R^1$ in each case is H, formyl or acetyl and $R^2$ in each case is H or $CH_3$, characterised in that a steroid of the general formula II

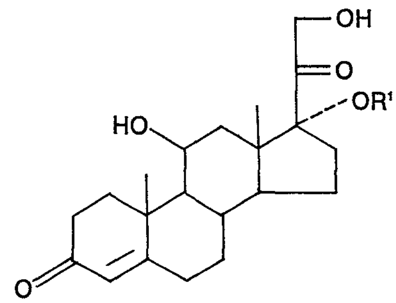

II

in which the radical $R^1$ is as defined above or a functionally modified derivative thereof, is reacted with a 2-hydroxy-fatty acid of the general formula III

$CH_3$-$CR^2$(OH)-COOH III

in which the radical $R^2$ is as defined above, or with a functionally modified derivative thereof, and in that, if a hydroxyl group is present in the substituent in the 21-position, this group is acetylated.

**Revendications pour les Etats contractants:**
BE, CH, DE, FR, GB, IT, NL, SE

1. Esters d'hydrocortisone de formule générale I

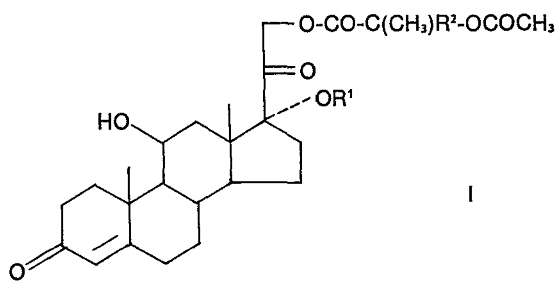

I

où $R^1$ représente à chaque fois un atome d'hydrogène, un groupe formyle ou acétyle et
$R^2$ représente à chaque fois un atome d'hydrogène ou un groupe $CH_3$.

2. a) 21-(2-acétoxypropionate) d'hydrocortisone
   b) 17-acétate-21-(2-acétoxypropionate) d'hydrocortisone
   c) 21-(2-acétoxyisobutyrate) d'hydrocortisone
   d) 17-acétate-21-(2-acétoxyisobutyrate) d'hydrocortisone.

3. Procédé de préparation d'esters d'hydrocortisone de formule générale I, caractérisé en ce qu'on fait réagir un stéroïde de formule générale II

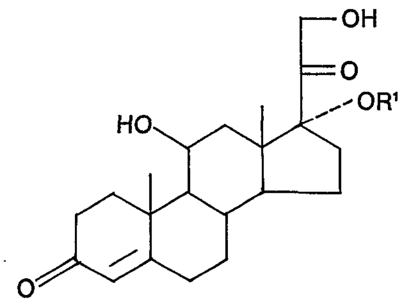

II

où le radical $R^1$ a la signification donnée dans la revendication 1, ou un de ses dérivés fonctionnellement modifiés, avec un acide gras 2-hydroxylé de formule générale III

$CH_3$-$CR^2$(OH)-COOH III

où le radical $R^2$ a la signification donnée dans la revendication 1, ou un de ses dérivés fonctionnellement modifiés, et en ce qu'on acétyle ceux-ci si un groupe hydroxy est présent dans le substituant en 21.

4. Procédé de préparation de préparations pharmaceutiques, caractérisé en ce qu'on amène sous une forme posologique appropriée un composé de formule I avec au moins un support ou additif solide, liquide ou semi-liquide et éventuellement en combinaison avec une autre substance active.

5. Préparation pharmaceutique caractérisée en ce qu'elle contient au moins un composé de formule générale I.

**Revendication pour l'Etat contractant:** AT

Procédé de préparation d'esters d'hydrocortisone de formule générale I

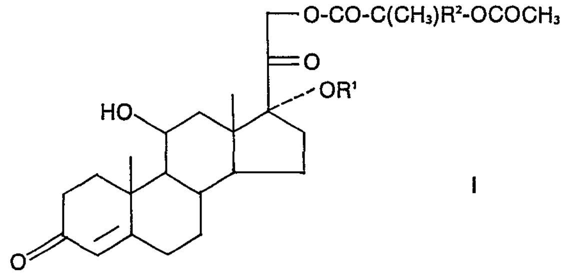

I

où $R^1$ représente à chaque fois un atome d'hydrogène, un groupe formyle ou acétyle et
$R^2$ représente à chaque fois un atome d'hydrogène ou un groupe $CH_3$,

caractérisé en ce qu'on fait réagir un stéroïde de formule générale II

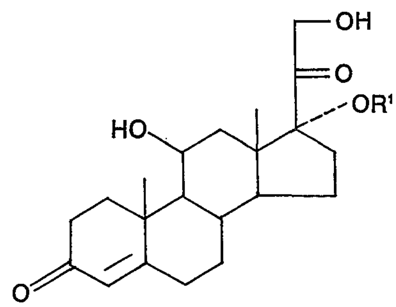

II

où le radical $R^1$ a la signification donnée ci-dessus, ou un de ses dérivés fonctionnellement modifiés, avec un acide gras 2-hydroxylé de formule générale III

$CH_3$-$CR^2$(OH)-COOH III

où le radical $R^2$ a la signification donnée ci-dessus, ou un de ses dérivés fonctionnellement modifiés, et en ce qu'on acétyle ceux-ci si un groupe hydroxy est présent dans le substituant en 21.